# EUROPÄISCHE PATENTSCHRIFT

(11) **EP 1 406 546 B1**
(45) Veröffentlichungstag und Bekanntmachung des Hinweises auf die Patenterteilung: **15.12.2004**
(21) Anmeldenummer: 03732379.7
(22) Anmeldetag: 15.05.2003
(51) Int. Cl.: A61B 17/28

(54) **MEDIZINISCHES INSTRUMENT**
MEDICAL INSTRUMENT
INSTRUMENT MEDICAL

(30) Priorität: 01.06.2002 DE 10224336
(43) Veröffentlichungstag der Anmeldung: 14.04.2004
(73) Patentinhaber: The University of Dundee, Dundee DD1 4HN (GB)
(72) Erfinder: FRANK, Tim, Wormit, Fife KY16 9TY (GB); CUSCHIERI, Alfred, St. Andrews, Fife KY16 9TY (GB); MARTIN, Duncan, Dundee DD2 2EZ (GB); GOVE, James, Dundee DD4 6LS (GB)
(74) Vertreter: Hofmeister, Frank Horst
(86) Internationale Anmeldenummer: PCT/EP2003/005083
(87) Internationale Veröffentlichungsnummer: WO 2003/101316

(56) Entgegenhaltungen:
- DE-A- 4 334 746
- DE-C- 19 912 038
- US-A- 4 569 131
- US-A- 4 950 273

## Beschreibung

Die Erfindung betrifft ein medizinisches Instrument, insbesondere endoskopisches Instrument, mit einem Instrumentenschaft, einem am distalen Ende des instrumentenschaftes angeordneten Werkzeug sowie einer Handhabe, die über ein Kopplungselement in axialer Erstreckung des Instrumentenschaftes verlagerbar am Instrumentenschaft festlegbar ist.

insbesondere bei Instrumenten für die laparoskopische Chirurgie weisen die medizinischen Instrumente häufig Schaftlängen von 30 cm und mehr auf. Diese Schaftlänge stellt sicher, dass auch weiter entfernt gelegene Operationsgebiete erreicht werden können, ohne den Instrumentenzugang verlegen zu müssen. Da die Instrumente jedoch die meiste Zeit für nahe gelegene Operationsgebiete verwendet werden, befindet sich der längste Teil des Instrumentenschaftes außerhalb des Körpers des Patienten. Daraus kann eine für den Operateur ungünstige und unbequeme Haltung bzw. Arbeitsstellung resultieren, da die Handhabe zum Führen und/oder Betätigen des chirurgischen Instruments am proximalen Ende des Instrumentenschafts angeordnet ist.

Dieses Problem wird bei der HALS Operationstechnik (Hand Assisted Laparoscopic Surgery) noch verstärkt, bei der zusätzlich zum Einbringen des Laparoskops und gegebenenfalls laparoskopischer Instrumente in die Bauchhöhle ein Hautschnitt zum Einführen einer Hand des Operateurs geschaffen wird, damit der Operateur per Tastsinn und unter Beobachtung und Kontrolle durch das Laparoskop eine besser geführte Operation durchführen kann. Während der Operateur mit einer Hand in der Bauchhöhle des Patienten die Operation unterstützt, betätigt er mit der anderen Hand die laparoskopischen Instrumente. Eine weit entfernt am Ende des Instrumentenschaftes angeordnete Handhabe erschwert die Arbeit des Operateurs dabei nicht unerheblich.

Aus der DE 44 13 520 A1 ist eine Vorrichtung zum Platzieren von Trokaren oder Punktionskanülen bekannt. Diese Vorrichtung besteht im wesentlichen aus einer zwei Griffteile aufweisenden Handhabe und einem in dem Handhabengehäuse angeordneten Transportmechanismus, über den der in das Handhabengehäuse eingesteckte Instrumentenschaft eines Trokars oder einer Punktionskanüle in Richtung der Punktionsstelle verlagerbar ist.

Der eigentliche Transportmechanismus, über den der Instrumentenschaft durch das Handhabengehäuse transportierbar ist, besteht bei dieser bekannten Vorrichtung gemäß einer ersten Ausführungsform aus einem federbelasteten Klemmmechanismus und gemäß einer zweiten Ausführungsform aus einer Verzahnung zwischen einem Griffteil der Handhabe und dem Instrumentenschaft.

Dieses bekannte Vorrichtung weist den Nachteil auf, dass die Handhabe bei der Ausführungsform mit dem verzahnten Transportmechanismus ausschließlich in Längsrichtung des Instrumentenschaftes am Instrumentenschaft festlegbar. Bei der Ausgestaltung des Transportmechanismus mit dem federbelasteten Klemmmechanismus besteht zumindest theoretisch die Möglichkeit, die Handhabe zusätzlich um die Längsachse des Instrumentenschaftes zu drehen, so dass in diesem Fall maximal zwei Freiheitsgrade zur Anordnung der Handhabe bezüglich des Instrumentenschaftes zur Verfügung stehen.

Um dem Operateur eine jederzeit sichere und bequeme Handhabung eines medizinischen Instruments zu gewährleisten, ist eine solchermaßen beschränkte Verstellbarkeit der Handhabe aber nicht ausreichend.

Weiterhin ist aus der DE 199 12 038 C1 ein medizinisches Instrument bekannt, dessen Handhabe gegenüber dem Instrumentenschaft verschwenkbar ist, um dem Operateur eine stets passende Winkelstellung zum Ergreifen der Handhabe zu ermöglichen. Diese verschwenkbare Handhabe erleichtert die Arbeit des Operateurs bei ungünstigen Platzverhältnissen zwar ganz erheblich, jedoch lassen sich ungünstige Arbeitsstellungen, die in erster Linie aus der Länge des Instrumentenschafts resultieren, durch diese Verschwenkbarkeit nicht, oder nur unzureichend beheben.

Davon ausgehend liegt der Erfindung die **Aufgabe** zugrunde, ein medizinisches Instrument der eingangs genannten Art zu schaffen, dass auch bei den unterschiedlichsten Verwendungszwecken eine bequeme, sichere und zuverlässige Handhabung ermöglicht.

Die **Lösung** dieser Aufgabenstellung ist erfindungsgemäß dadurch gekennzeichnet, dass die Handhabe um mindestens drei Freiheitsgrade gegenüber dem Instrumentenschaft verstellbar am Instrumentenschaft festlegbar ist.

Durch die erfindungsgemäße Ausgestaltung, der entlang der Längserstreckung des Schaftes verlagerbar festlegbaren Handhabe besteht nunmehr für den Operateur die Möglichkeit, die Handhabe je individuell angepaßt am Instrumentenschaft anzuordnen, so dass sich für jeden Operationsabschnitt eine bestmögliche Lage der Handhabe ergibt, um das medizinische Instrument bequem und sicher zu führen.

Gemäß einer praktischen Ausführungsform der Erfindung ist das Kopplungselement als den Instrumentenschaft zumindest teilweise umschließendes und mit dem Instrumentenschaft verklemmbares Bauteil ausgebildet. Das Verklemmen des Kopplungselement mir dem Instrumentenschaft stellt sicher, dass die Handhabe ortsfest am Instrumentenschaft festlegbar ist und eine auf die Handhabe ausgeübte Bewegung auch auf den Instrumentenschaft übertragen werden kann.

Bei einer bevorzugten Ausführungsform der Erfindung wird vorgeschlagen, dass das distale Ende der Handhabe als Spannvorrichtung zur Aufnahme des Kopplungselements ausgebildet ist, wobei Ober die Spannvorrichtung eine Druckkraft derart auf das Kopplungselement ausübbar ist, dass das Kopplungselement den Instrumentenschaft zumindest teilweise klemmend umschließt.

Um ein Kopplungselement zu schaffen, dass einen sichern Halt an und auf dem Instrumentenschaft gewährleistet und darüber hinaus einfach und kostengünstig zu fertigen ist, wird mit der Erfindung vorgeschlagen, dass das Kopplungselement als mit einer Durchgangsbohrung für den Instrumentenschaft versehenes, im wesentlichen zylindrisches oder kugelförmiges Bauteil ausgebildet ist und ist die Spannvorrichtung der Handhabe zur verdrehbaren Lagerung des Kopplungselements als Lagerschale ausgebildet.

Bei dieser Ausgestaltung bilden das Kopplungselement und die Spannvorrichtung eine Art Kugelgelenk, wodurch ein zusätzliches Verschwenken und Kippen der Handhabe relativ zum tnstrumentenschaft möglich ist. Zusammen mit der Längsverlagerung der Handhabe entlang dem Instrumentenschaft sowie der Rotation des Instrumentenschafts ergeben sich somit bei dieser erfindungsgemäßen Ausgestaltungsform vier Freiheitsgrade für die an dem Instrumentenschaft gelagerte Handhabe, nämlich eine translatorische Bewegung in Richtung der Längsachse des Instrumentenschaftes und drei rotatorische Bewegungen, eine um die Längsachse des Instrumentenschaftes und zwei um eine Achse senkrecht zur Längsachse des Instrumentenschaftes.

Bei einer alternativen Ausführungsform der Erfindung wird vorgeschlagen, dass das als kugelförmiges Bauteil ausgebildete Kopplungselement zumindest einseitig einen vom Außenumfang bis zur Durchgangsbohrung verlaufenden, in Axialrichtung des lnstrumentenschaftes ausgebildeten Schnitt aufweist.

Weiterhin wird mit der Erfindung vorgeschlagen, dass das kugelförmige Kopplungselement aus mindestens zwei in Axialrichtung des Instrumentenschaftes geteilten Kugelsegmenten besteht. Die Ausbildung des Kopplungselements beispielsweise aus zwei Halbkugeln ermöglicht eine einfache und schnelle Montage des Kopplungselements am Instrumentenschaft und erlaubt ein Verspannen des Kopplungselements auch bei der Verwendung harter Materialien.

Gemäß einer ersten Ausführungsform der Erfindung besteht das Koppiungselement dabei aus einem zusammendrückbaren Material, insbesondere einem Gummi- oder Kunststoffmaterial.

Eine zweite Ausführungsform der Erfindung erlaubt auch die Verwendung inkompressibler Materialien, wie beispielsweise hartem Kunststoff- oder metallenem Material, zur Ausbildung des Kopplungselements, da der mindestens eine Schnitt ein Verklemmen des Kopplungselements mit dem Instrumentenschaft durch das Aufbringen einer äußeren Druckkraft ermöglicht.

Um eine einfach zu fertigende und sicher zu handhabende Handhabe zu schaffen, wird mit der Erfindung vorgeschlagen, dass die Handhabe proximalseitig zwei Handgriffe aufweist, wobei wenigstens ein Handgriff um eine Schwenkachse relativ zu dem anderen Handgriff verschwenkbar gelagert ist.

Damit der Operateur die Handgriffe der Handhabe Handhabe nicht permanent zusammendrücken muß, um die Handhabe ortsfest am Instrumentenschaft zu halten, ist die Handhabe in einer Schließstellung, in der das Kopplungselement mit dem Instrumentenschaft verklemmt ist, arretierbar, wozu zum Arretieren der Handhabe in der Schließstellung an der Handhabe eine Arretiervorrichtung angeordnet ist, die vorzugsweise als im Bereich der Spannvorrichtung angeordnete Gewindeverschraubung oder als Exzenterverschluß ausgebildet ist.

Weiterhin wird mit der Erfindung vorgeschlagen, dass die verdrehbewegliche Lagerung des Kopplungselements in der Spannvorrichtung über einen Sperrstift begrenzbar ist, so dass die Handhabe ortsfest und lagegenau relativ zum lnstrumentenschaft am Instrumentenschaft fixierbar ist.

Bei einer weiteren praktischen Ausführungsform der Erfindung wird vorgeschlagen, dass bei einem medizinischen Instrument mit einem am distalen Ende des instrumentenschaftes angeordneten Werkzeug das Werkzeug Ober die Handgriffe der Handhabe betätigbar ist, wobei die Handhabe und das Werkzeug Ober mindestens eine Kraftübertragungsvorrichtung miteinander verbunden sind. Die Kraftübertragungsvorrichtung ist dabei vorzugsweise als flexibles Kraftübertragungselement, insbesondere als Bowdenzug, oder hydraulisch arbeitende Kraftübertragungsvorrichtung ausgebildet.

Gemäß einer bevorzugten Ausführungsform der Erfindung kann zusätzlich zur Betätigung des Werkzeugs über die Handgriffe der Handhabe auch die Spannvorrichtung Ober die Handgriffe der Handhabe betätigbar sein. Bei dieser Ausführungsform ist es vorteilhaft, wenn die Betätigung der Spannvorrichtung über die Handgriffe der Handhabe von der Betätigung des Werkzeugs über die Handgriffe der Handhabe entkoppelbar ist.

Diese Entkopplung der Betätigungsfunktionen der Handgriffe ist besonders wirksam, wenn gleichzeitig mit der Auswahl einer Betätigungsfunktion automatisch die andere Betätigungsfunktion gesperrt wird, wozu an der Handhabe eine Umschaltvorrichtung zur Einstellung der über die Handgriffe der Handhabe ausübbaren Betätigungsfunktion angeordnet ist.

Weitere Merkmale und Vorteile der Erfindung ergeben sich anhand der nachfolgenden Beschreibung der zugehörigen Zeichnung, in der ein Ausführungsbeispiel eines erfindungsgemäßen medizinischen Instruments nur beispielhaft schematisch dargestellt ist. In der Zeichnung zeigt:
- Fig. 1: eine Seitenansicht einer ersten Ausführungsform eines erfindungsgemäßen medizinischen Instruments und
- Fig. 2: eine Rückansicht des medizinischen Instruments gemäß Fig. 1.

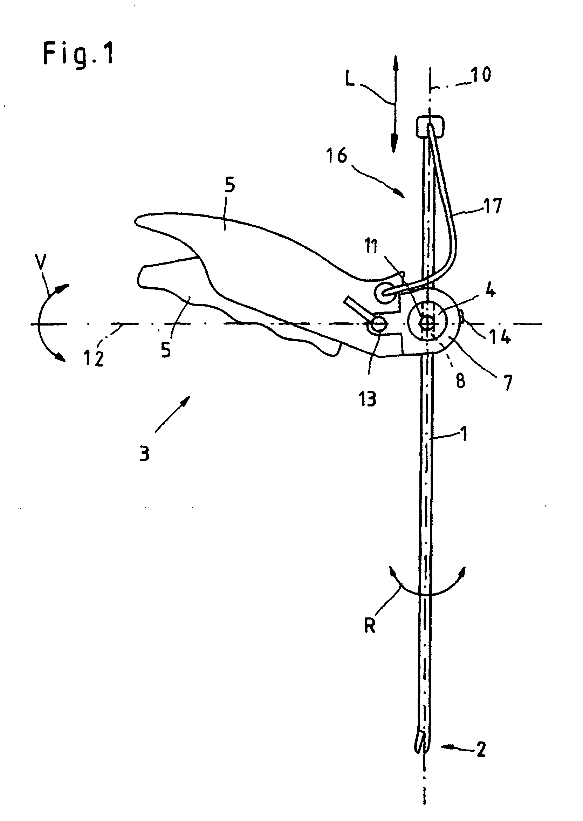

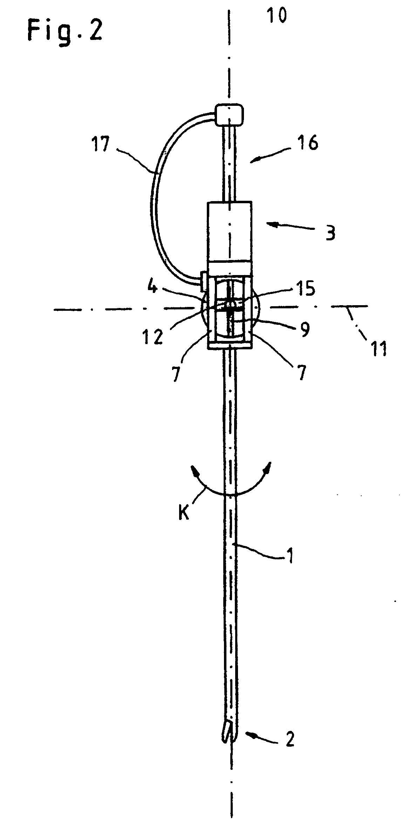

Das in den Abbildungen Fig. 1 und 2 dargestellte medizinische Instrument besteht im wesentlichen aus einem langgestreckten zylindrischen Instrumentenschaft 1, einem am distalen Ende des Instrumentenschaftes 1 angeordneten, als Greifwerkzeug ausgebildeten Werkzeug 2 sowie einer Handhabe 3 zum Handhaben des Instrumentenschaftes 1 und/oder des Greifwerkzeugs 2.

Hervorstechend bei dem dargestellten medizinischen Instrument ist, dass die Handhabe 3 nicht, wie bei den aus dem Stand der Technik bekannten medizinischen Instrumenten üblich, ortsfest am proximalen Ende des Instrumentenschaftes 1 angeordnet ist, sondern statt dessen in axialer Erstreckung des Instrumentenschaftes 1 an beliebigen Stellen des Instrumentenschaftes 1 klemmend festlegbar ist.

Das klemmende Festlegen der Handhabe 3 am Instrumentenschaft 1 erfolgt Ober ein Kopplungselement 4, das als den Instrumentenschaft 1 zumindest teilweise umschließendes Bauteil ausgebildet ist. Bei der in den Abbildungen dargestellten

Ausführungsform ist das Kopplungselement 4 als kugelförmiges Bauteil ausgebildet. Selbstverständlich ist es auch möglich, das Kopplungselement 4 beispielsweise als den Instrumentenschaft 1 zumindest teilweise umschließendes zylindrisches Bauteil auszugestalten.

Wie aus den Abbildungen weiterhin ersichtlich, weist die verstellbar am lnstrumentenschaft 1 festlegbare Handhabe 3 bei der dargestellten Ausführungsform eines medizinischen Instruments proximalseitig zwei Handgriffe 5 auf, von denen wenigstens einer um eine Schwenkachse 6 relativ zu dem anderen Handgriff 5 verschwenkbar ist. Das distalseitige Ende der Handhabe 3 bildet eine Spannvorrichtung 7, die zur Aufnahme des Kopplungselements 4 dient.

Das mit einer Durchgangsbohrung 8 für den Instrumentenschaft 1 versehene kugelförmige Kopplungselement 4 besteht gemäß den Abbildungen Fig. 1 und 2 aus zwei in Axialrichtung des Instrumentenschaftes 1 geteilten halbkugelförmigen Kugelsegmenten, die in der als Lagerschale ausgebildeten Spannvorrichtung 7 der Handhabe 3 verdrehbar gelagert sind.

Über die Spannvorrichtung 7 ist eine Druckkraft auf das Kopptungselement 4 ausübbar, die bewirkt, dass das Kopplungselement 4 den Instrumentenschaft 1 klemmend umschließt. Wenn das Kopplungselement 4 aus einem zusammendrückbaren Material, wie beispielsweise einem Gummi- oder Kunststoffmaterial, besteht, ist die Verformbarkeit des Materials ausreichend, um über die Druckkraft eine kraftschlüssige Verbindung zwischen der Handhabe 3 und dem Instrumentenschaft 1 zu erzeugen, die eine ortsfeste und lagegenaue Fixierung der Handhabe 3 am Instrumentenschaft 1 gewährleistet.

Besteht dahingegen das Kopplungselement 4 aus einem inkompressiblen oder einem nicht elastisch verformbaren Material, wie beispielsweise einem harten Kunststoff oder einem metallenen Material, weist das Kopptungselement 4 mindestens einen vom Außenumfang bis zur Durchgangsbohrung 8 verlaufenden, in Axialrichtung des Instrumentenschaftes 1 ausgebildeten Schnitt 9 auf. Beim Aufbringen der Druckkraft über die Spannvorrichtung 7 wird dieser Schnitt 9 geschlossen und so das Kopplungselement 4 kraftschlüssig mit dem Instrumentenschaft 1 verklemmt wird.

Bei der Ausbildung des Kopplungselements 4 als aus zwei halbkugelförmigen Kugelsegmenten bestehend, weist das Kopplungselement 4 somit zwei um 180° zueinander versetzt angeordnete Schnitte 9 auf.

Aufgrund der beschriebenen Ausbildung der Handhabe 3, die Ober das Kopplungselement 4 am instrumentenschaft 1 festlegbar ist, hat der Operateur die Möglichkeit, die Lage der Handhabe 3 frei zu bestimmen. Befindet sich das Operationsgebiet nahe der Eintrittsstelle in den Körper des Patienten, kann er die Handhabe 3 weit zum distalen Ende des Instrumentenschaftes 1 versetzt anordnen, um eine ungünstige Arbeitshaltung zu vermeiden. Wird das medizinische Instrument dahingegen für ein weiter von der Eintrittsstelle entferntes Operationsgebiet benötigt, läßt sich die Handhabe 3 schnell und einfach zum proximalen Ende hin verlagern.

Neben der Möglichkeit, die Handhabe 3 translatorisch in Richtung der Längsachse 10 des Instrumentenschaftes 1 zu verlagern, ist die Handhabe 3 rotatorisch um die Längsachse 10 des Instrumentenschaftes 1 verdrehbar, so dass bei diesem prinzipiellen Aufbau der am instrumentenschaft 1 festlegbaren Handhabe 3 die Handhabe 3 mindestens zwei Freiheitsgrade bezüglich der Längsachse 10 des Instrumentenschaftes 1 aufweist, nämlich einen für eine translatorische Bewegung und einen für eine rotatorische Bewegung.

Bei der in den Abbildungen Fig. 1 und 2 dargestellten Ausführungsform, bei der das Kopplungselement 4 als kugelförmiges Bauteil ausgebildet ist, das ein Art Kugelgelenk bildend in der Lagerschale der Spannvorrichtung 7 verdrehbar gelagert ist, kommen noch zwei weitere rotatorische Freiheitsgrade für die Bewegung der Handhabe 3 gegenüber dem Instrumentenschaft 1 hinzu. Diese zwei weiteren Freiheitsgrade sind einerseits die Verschwenkbarkeit der Handhabe 3 um die senkrecht zur Längsachse 10 des Instrumentenschaftes 1 verlaufende Achse 11 gemäß Fig. 1 sowie andererseits die kippbewegliche Lagerung der Handhabe 3 um die senkrecht zur Längsachse 10 des Instrumentenschaftes 1 verlaufende Achse 12 gemäß Fig. 2.

Eine solchermaßen ausgestaltete Handhabe 3 zeichnet sich somit dadurch aus, dass sie insgesamt vier Freiheitsgrade besitzt, um die sie gegenüber dem lnstrumentenschaft 1 verstellbar ist, so dass die Handhabe 3 einfach und schnell in jede vom Operateur gewünschte Position überführbar ist, um eine bequeme, sichere und zuverlässige Handhabung des medizinischen Instruments zu gewährleisten.

Um sicherzustellen, dass die Handhabe 3 in der einmal gewählten Position am Instrumentenschaft 1 ortsfest und lagegenau verbleibt, ist die Handhabe 3 in der am instrumentenschaft 1 verklemmten Schließstellung über einen Arretiervorrichtung 13 arretierbar. Zusätzlich ist die verdrehbewegliche Lagerung des Kopptungselements 4 in der Spannvorrichtung 7 über einen im Bereich der Spannvorrichtung 7 angeordneten Sperrstift 14 begrenzbar. Sobald somit über die Arretiervorrichtung 13 sichergestellt ist, dass das Kopplungselement 4 mit dem Instrumentenschaft 1 verklemmt ist, kann der Operateur das medizinische Instrument über die Handhabe 3 sicher und bequem führen, da die Verstellbarkeit der Spannvorrichtung 7 nunmehr blockiert ist.

Bei der in Fig. 1 und 2 dargestellten Ausführungsform besteht die Arretiervorrichtung 13 aus einer Gewindeverschraubung 15, über die, wie aus Fig. 2 ersichtlich, die beiden Seitenwände der Spannvorrichtung 7 so aufeinander zu zusammengezogen werden, dass die Kugelsegmente des Kopplungselements 4 verklemmend gegen den Instrumentenschaft 1 gedrückt werden.

Alternativ zur Verwendung einer Gewindeverschraubung 15 zur Ausbildung der Arretiervorrichtung 13 sind selbstverständlich auch andere geeignete Maßnahmen, wie beispielsweise die Ausbildung eines Exzenterverschlusses, möglich, um über die Spannvorrichtung 7 eine dauerhafte Druckkraft auf das Kopplungselement 4 auszuüben, so dass die Handhabe ortsfest und lagegenau am Instrumentenschaft 1 gehalten wird.

Bei dem in den Abbildungen Fig. 1 und 2 dargestellten medizinischen Instrument ist am distalen Ende des Instrumentenschaftes 1 ein Werkzeug 2 angeordnet. Zum Bedienen dieses Greifwerkzeugs 2 über die Handhabe 3 ist die Handhabe 3 mit dem Greifwerkzeug 2 über ein Kraftübertragungselement 16 verbunden, das vorzugsweise durch den hohlen Instrumentenschaft 1 geführt ist. Das Betätigen des Greifwerkzeugs 2 erfolgt über die Handgriffe 5 der Handhabe 3. Bei diesem in Fig. 1 und 2 dargestellten medizinischen Instrument ist das Kraftübertragungselement 16 als Bowdenzug 17 ausgebildet. Bei der Ausbildung des Kraftübertragungselement 16 als Bowdenzug 17 ist es zum Öffnen des distalseitigen Werkzeugs 2 erforderlich, eine nicht dargestellte Rückstellfeder vorzusehen.

Es sind jedoch auch andere Kraftübertragungselemente 16, wie beispielsweise die Verwendung eines hydraulisch arbeitenden Kraftübertragungselements, möglich.

Selbstverständlich ist eine wie voranstehend beschrieben ausgebildete Handhabe 3, die an beliebiger Stelle an einem Instrumentenschaft 1 festlegbar ist, auch für andere medizinische Instrumente verwendbar, bei denen nicht gleichzeitig über die Handhabe 3 ein Werkzeug in oder am Instrumentenschaft 1 betätigt wird. Bei diesen Ausgestaltungsformen entfällt dann das mit der Handhabe 3 verbundene Kraftübertragungselement.

Das Bedienen eines gemäß den Abbildungen Fig. 1 und 2 ausgebildeten medizinischen Instruments geschieht wie folgt:

In der in Fig. 1 dargestellten Arbeitsstellung ist die Handhabe 3 ortsfest und lagegenau am instrumentenschaft 1 so verklemmt, dass der Operateur das medizinische Instrument und insbesondere das am distalen Ende des Instrumentenschaftes 1angeordnete Werkzeug 2 über die Handgriffe 5 der Handhabe 3 bequem und sicher bedienen kann.

Soll nun beispielsweise an einem weiter von der Eintrittsstelle des Instruments in den Körper des Patienten entfernten Operationsgebiet gearbeitet werden, kann der Operateur die Handhabe 3 weiter zum proximalen Ende des Instrumentenschaftes 1 hin verlagern. Zu diesem Zweck betätigt der Operateur die Arretiervorrichtung 13 sowie den Sperrstift 14, so dass die Handhabe 3 nunmehr die zur Verfügung stehenden vier Freiheitsgrade ausnutzend gegenüber dem Instrumentenschaft 1 verstellt werden kann. Sobald die Handhabe 3 sich in der für den Operateur optimalen Stellung befindet, werden die Arretiervorrichtung 13 sowie der Sperrstift 14 erneut betätigt, um die Handhabe 3 wieder ortsfest und lagegenau am Instrumentenschaft 1 zu fixieren.

In Fig. 1 sind drei Freiheitsgrade der Verstellbarkeit der Handhabe 3 gegenüber dem Instrumentenschaft 1 dargestellt, nämlich die translatorische Bewegung in Richtung der Längsachse 10 des Instrumentenschaftes 1 mit dem Pfeil L, die Rotation um die Längsachse 10 des Instrumentenschaftes 1 mit dem Pfeil R sowie die rotatorische Verschwenkbarkeit um die senkrecht zur Längsachse 10 des Instrumentenschaftes 1 verlaufende Achse 11 mit dem Pfeil V.

Der mögliche vierte Freiheitsgrad ist in Fig. 2 dargestellt und zeigt mit dem Pfeil K die rotatorische Kippbeweglichkeit der Handhabe 3 um die senkrecht zur Längsachse 10 des Instrumentenschaftes 1 verlaufende Achse 12.

### Bezugszeichenliste

- 1: lnstrumentenschaft
- 2: (Greif-)Werkzeug
- 3: Handhabe
- 4: Kopplungselement
- 5: Handgriff
- 6: Schwenkachse
- 7: Spannvorrichtung
- 8: Durchgangsbohrung
- 9: Schnitt
- 10: Längsachse
- 11: Achse
- 12: Achse
- 13: Arretiervorrichtung
- 14: Sperrstift
- 15: Gewindeverschraubung
- 16: Kraftübertragungselement
- 17: Bowdenzug

- L: Pfeil (Längsbeweglichkeit)
- K: Pfeil (Kippbeweglichkeit)
- R: Pfeil (Rotation)
- V: Pfeil (Verschwenkbarkeit)

## Patentansprüche

1. Medizinisches Instrument, insbesondere endoskopisches Instrument, mit einem Instrumentenschaft (1), einem am distalen Ende des Instrumentenschaftes (1) angeordneten Werkzeug (2) sowie einer Handhabe (3), die über ein Kopplungselement (4) in axialer Erstreckung des Instrumentenschaftes (1) verlagerbar am Instrumentenschaft (1) festlegbar ist,
**dadurch gekennzeichnet,**
**dass** die Handhabe (3) um mindestens drei Freiheitsgrade gegenüber dem Instrumentenschaft (1) verstellbar am Instrumentenschaft (1) festlegbar ist.

2. Medizinisches Instrument nach Anspruch 1, **dadurch gekennzeichnet, dass** das Kopplungselement (4) als den Instrumentenschaft (1) zumindest teilweise umschließendes und mit dem Instrumentenschaft (1) verklemmbares Bauteil ausgebildet ist.

3. Medizinisches Instrument nach Anspruch 1 oder 2, **dadurch gekennzeichnet, dass** das distale Ende der Handhabe (3) als Spannvorrichtung (7) zur Aufnahme des Kopplungselements (4) ausgebildet ist.

4. Medizinisches instrument nach Anspruch 3, **dadurch gekennzeichnet, dass** über die Spannvorrichtung (7) eine Druckkraft derart auf das Kopplungselement (4) ausübbar ist, dass das Kopplungselement (4) den Instrumentenschaft (1) zumindest teilweise klemmend umschließt.

5. Medizinisches Instrument nach mindestens einem der Ansprüche 2 bis 4, **dadurch gekennzeichnet, dass** das Kopplungselement (4) als mit einer Durchgangsbohrung (8) für den Instrumentenschaft (1) versehenes, im wesentlichen kugelförmiges Bauteil ausgebildet ist und die Spannvorrichtung (7) der Handhabe (3) als Lagerschale zur verdrehbaren Lagerung des Kopplungselements (4) ausgebildet ist.

6. Medizinisches Instrument nach Anspruch 5, **dadurch gekennzeichnet, dass** das als kugelförmiges Bauteil ausgebildete Kopplungselement (4) zumindest einseitig einen vom Außenumfang bis zur Durchgangsbohrung (8) verlaufenden, in Axialrichtung des Instrumentenschaftes (1) ausgebildeten Schnitt (9) aufweist.

7. Medizinisches Instrument nach Anspruch 5, **dadurch gekennzeichnet, dass** das kugelförmige Kopplungselement (4) aus mindestens zwei in Axialrichtung des Instrumentenschaftes (1) geteilten Kugelsegmenten besteht.

8. Medizinisches Instrument nach mindestens einem der Ansprüche 2 bis 7, **dadurch gekennzeichnet, dass** das Kopplungselement (4) aus einem zusammendrückbaren Material, insbesondere einem Gummi- oder Kunststoffmaterial, besteht.

9. Medizinisches Instrument nach Anspruch 6 oder 7, **dadurch gekennzeichnet, dass** das Kopplungselement (4) aus einem inkompressiblen Material, insbesondere einem harten Kunststoff- oder metallenen Material, besteht.

10. Medizinisches Instrument nach mindestens einem der Ansprüche 1 bis 9, **dadurch gekennzeichnet, dass** die Handhabe (3) proximalseitig zwei Handgriffe (5) aufweist, wobei wenigstens ein Handgriff (5) um eine Schwenkachse (6) relativ zu dem anderen Handgriff (5) verschwenkbar gelagert ist.

11. Medizinisches Instrument nach mindestens einem der Ansprüche 3 bis 10, **dadurch gekennzeichnet, dass** die Handhabe (3) in einer Schließstellung, in der das Kopplungselement (4) mit dem Instrumentenschaft (1) verklemmt ist, arretierbar ist.

12. Medizinisches Instrument nach Anspruch 11, **dadurch gekennzeichnet, dass** zum Arretieren der Handhabe (3) in der Schließstellung an der Handhabe (3) ein Arretiervorrichtung (13) angeordnet ist.

13. Medizinisches Instrument nach Anspruch 12, **dadurch gekennzeichnet, dass** die Arretiervorrichtung (13) als Gewindeverschraubung (15) im Bereich der Spannvorrichtung (7) ausgebildet ist.

14. Medizinisches Instrument nach Anspruch 12, **dadurch gekennzeichnet, dass** die Arretiervorrichtung (13) als Exzenterverschluß im Bereich der Spannvorrichtung (7) ausgebildet ist.

15. Medizinisches Instrument nach mindestens einem der Ansprüche 5 bis 14, **dadurch gekennzeichnet, dass** die verdrehbewegliche Lagerung des Kopplungselements (4) in der Spannvorrichtung (7) über einen Sperrstift (14) begrenzbar ist.

16. Medizinisches Instrument nach mindestens einem der Ansprüche 1 bis 15 mit einem am distalen Ende des Instrumentenschaftes (1) angeordneten Werkzeug, das über die Handhabe (3) betätigbar ist, **dadurch gekennzeichnet, dass** das Werkzeug (2) über die Handgriffe (5) der Handhabe (3) betätigbar ist, wobei die Handhabe (3) und das Werkzeug (2) über mindestens eine Kraftübertragungsvorrichtung (16) miteinander verbunden sind.

17. Medizinisches Instrument nach Anspruch 16, **dadurch gekennzeichnet, dass** die mindestens eine Kraftübertragungsvorrichtung (16) als flexibles Kraftübertragungselement, insbesondere als Bowdenzug (17), ausgebildet ist.

18. Medizinisches Instrument nach Anspruch 16, **dadurch gekennzeichnet, dass** die mindestens eine Kraftübertragungsvorrichtung (16) hydraulisch arbeitet.

19. Medizinisches Instrument nach mindestens einem der Ansprüche 16 bis 18, **dadurch gekennzeichnet, dass** zusätzlich zur Betätigung des Werkzeugs (2) über die Handgriffe (5) der Handhabe (3) auch die Spannvorrichtung (7) über die Handgriffe (5) der Handhabe (3) betätigbar ist.

20. Medizinisches Instrument nach Anspruch 19, **dadurch gekennzeichnet, dass** die Betätigung der Spannvorrichtung (7) über die Handgriffe (5) der Handhabe (3) von der Betätigung des Werkzeugs (2) über die Handgriffe (5) der Handhabe (3) entkoppelbar ist.

21. Medizinisches Instrument nach Anspruch 20, **dadurch gekennzeichnet, dass** die beiden über die Handgriffe (5) der Handhabe (3) ausübbaren Betätigungsfunktionen derart entkoppelbar sind, dass bei der Auswahl einer Betätigungsfunktion automatisch die andere Betätigungsfunktion gesperrt ist.

22. Medizinisches Instrument nach Anspruch 21, **dadurch gekennzeichnet, dass** an der Handhabe (3) eine Umschaltvorrichtung zur Einstellung der über die Handgriffe (5) der Handhabe (3) ausübbaren Betätigungsfunktion angeordnet ist.

## Claims

1. Medical instrument, in particular endoscopic instrument, with an instrument shaft (1), a tool (2) arranged at the distal end of the instrument shaft (1), and a handle (3) which can be secured on the instrument shaft (1) in such a way as to be displaceable along the axial extent of the instrument shaft (1) via a coupling element (4), **characterized in that** the handle (3) can be secured on the instrument shaft (1) so as to be adjustable about at least three degrees of freedom relative to the instrument shaft (1).

2. Medical instrument according to Claim 1, **characterized in that** the coupling element (4) is designed as a structural part which at least partially surrounds the instrument shaft (1) and which can be clamped on the instrument shaft (1).

3. Medical instrument according to Claim 1 or 2, **characterized in that** the distal end of the handle (3) is designed as a clamping device (7) for receiving the coupling element (4).

4. Medical instrument according to Claim 3, **characterized in that** a pressing force can be exerted on the coupling element (4) via the clamping device (7) so that the coupling element (4) at least partially surrounds the instrument shaft (1) with clamping.

5. Medical instrument according to at least one of Claims 2 to 4, **characterized in that** the coupling element (4) is designed as a substantially spherical structural part provided with a through-hole (8) for the instrument shaft (1), and the clamping device (7) of the handle (3) is designed as a bearing shell for pivotable bearing of the coupling element (4).

6. Medical instrument according to Claim 5, **characterized in that** the coupling element (4) designed as a spherical structural part has, at least at one end, an incision (9) which extends from the outer circumference to the through-hole (8) and which is formed in the axial direction of the instrument shaft (1).

7. Medical instrument according to Claim 5, **characterized in that** the spherical coupling element (4) is made up of at least two sphere segments divided in the axial direction of the instrument shaft (1).

8. Medical instrument according to at least one of Claims 2 to 7, **characterized in that** the coupling element (4) is made of a compressible material, in particular a rubber or plastic material.

9. Medical instrument according to Claim 6 or 7, **characterized in that** the coupling element (4) is made of a non-compressible material, in particular a hard plastic or metal material.

10. Medical instrument according to at least one of Claims 1 to 9, **characterized in that** the handle (3) has two handgrips (5) at the proximal end, at least one handgrip (5) being mounted so as to be able to pivot about a pivot axis (6) relative to the other handgrip (5).

11. Medical instrument according to at least one of Claims 3 to 10, **characterized in that** the handle (3) can be locked in a closed position in which the coupling element (4) is clamped on the instrument shaft (1).

12. Medical instrument according to Claim 11, **characterized in that** a locking device (13) is arranged on the handle (3) for the purpose of locking the handle (3) in the closed position.

13. Medical instrument according to Claim 12, **characterized in that** the locking device (13) is designed as a threaded screw connection (15) in the area of the clamping device (7).

14. Medical instrument according to Claim 12, **characterized in that** the locking device (13) is designed as an eccentric closure in the area of the clamping device (7).

15. Medical instrument according to at least one of Claims 5 to 14, **characterized in that** the pivoting movement allowed by the bearing of the coupling element (4) in the clamping device (7) can be limited via a blocking pin (14).

16. Medical instrument according to at least one of Claims 1 to 15, with a tool which is arranged at the distal end of the instrument shaft (1) and can be actuated via the handle (3), **characterized in that** the tool (2) can be actuated via the handgrips (5) of the handle (3), said handle (3) and said tool (2) being connected to one another via at least one force transmission device (16).

17. Medical instrument according to Claim 16, **characterized in that** the at least one force transmission device (16) is designed as a flexible force transmission element, in particular as a Bowden wire (17).

18. Medical instrument according to Claim 16, **characterized in that** the at least one force transmission device (16) operates hydraulically.

19. Medical instrument according to at least one of Claims 16 to 18, **characterized in that**, in addition to the tool (2) being actuated via the handgrips (5) of the handle (3), the clamping device (7) can also be actuated via the handgrips (5) of the handle (3).

20. Medical instrument according to Claim 19, **characterized in that** the actuation of the clamping device (7) via the handgrips (5) of the handle (3) can be decoupled from the actuation of the tool (2) via the handgrips (5) of the handle (3).

21. Medical instrument according to Claim 20, **characterized in that** the two actuation functions which can be performed via the handgrips (5) of the handle (3) can be decoupled in such a way that, when one actuating function is selected, the other actuating function is automatically blocked.

22. Medical instrument according to Claim 21, **characterized in that** a switch device is arranged on the handle (3) for the purpose of setting the actuating function that can be performed via the handgrips (5) of the handle (3).

## Revendications

1. Instrument médical, notamment instrument endoscopique, comprenant un corps d'instrument en tige (1), un outil (2) disposé à l'extrémité distale du corps d'instrument en tige (1), ainsi qu'une poignée de préhension (3) qui peut être fixée ou immobilisée par l'intermédiaire d'un élément de couplage (4) sur le corps d'instrument en tige (1) en pouvant être décalée sur l'étendue axiale du corps d'instrument en tige (1), **caractérisé en ce que** la poignée de préhension (3) peut être immobilisée ou fixée sur le corps d'instrument en tige (1) en étant réglable selon au moins trois degrés de liberté par rapport au corps d'instrument en tige (1).

2. Instrument médical selon la revendication 1, **caractérisé en ce que** l'élément de couplage (4) est réalisé en tant que pièce entourant au moins partiellement le corps d'instrument en tige (1), et pouvant être serrée par coincement sur le corps d'instrument en tige (1).

3. Instrument médical selon la revendication 1 ou 2, **caractérisé en ce que** l'extrémité distale de la poignée de préhension (3) est réalisée en tant que dispositif de serrage (7) pour recevoir l'élément de couplage (4).

4. Instrument médical selon la revendication 3, **caractérisé en ce que** par l'intermédiaire du dispositif de serrage (7), il est possible d'exercer une force de compression sur l'élément de couplage (4) de façon telle que l'élément de couplage (4) enserre par coincement au moins partiellement le corps d'instrument en tige (1).

5. Instrument médical selon l'une au moins des revendications 2 à 4, **caractérisé en ce que** l'élément de couplage (4) est réalisé en tant que pièce sensiblement de forme sphérique pourvue d'un alésage de passage (8) pour le corps d'instrument en tige (1), et le dispositif de serrage (7) de la poignée de préhension (3) est réalisé en tant que coque de palier pour le montage rotatif de l'élément de couplage (4).

6. Instrument médical selon la revendication 5, **caractérisé en ce que** l'élément de couplage (4) réalisé en tant que pièce de forme sphérique, présente au moins sur un côté, une incision ou fente (9) réalisée dans la direction axiale du corps d'instrument en tige (1) et s'étendant de la périphérie extérieure jusqu'à l'alésage de passage (8).

7. Instrument médical selon la revendication 5, **caractérisé en ce que** l'élément de couplage (4) de forme sphérique est formé d'au moins deux segments de sphère divisés dans la direction axiale du corps d'instrument en tige (1).

8. Instrument médical selon l'une au moins des revendications 2 à 7, **caractérisé en ce que** l'élément de couplage (4) est réalisé en un matériau compressible, notamment en un matériau du type caoutchouc ou matière plastique.

9. Instrument médical selon la revendication 6 ou 7, **caractérisé en ce que** l'élément de couplage (4) est réalisé en un matériau incompressible, notamment en une matière plastique dure ou un matériau métallique dur.

10. Instrument médical selon l'une au moins des revendications 1 à 9, **caractérisé en ce que** la poignée de préhension (3) présente, côté proximal, deux poignées de manoeuvre (5), au moins une poignée de manoeuvre (5) étant montée pivotante par rapport à l'autre poignée de manoeuvre (5), autour d'un axe de pivotement (6).

11. Instrument médical selon l'une au moins des revendications 3 à 10, **caractérisé en ce que** la poignée de préhension (3) peut être bloquée dans une position de fermeture dans laquelle l'élément de couplage (4) est serré par coincement sur le corps d'instrument en tige (1).

12. Instrument médical selon la revendication 11, **caractérisé en ce que** pour le blocage de la poignée de préhension (3) dans la position de fermeture, il est prévu un dispositif de blocage (13) sur la poignée de préhension (3).

13. Instrument médical selon la revendication 12, **caractérisé en ce que** le dispositif de blocage (13) est réalisé en tant que système de vissage fileté (15) dans la zone du dispositif de serrage (7).

14. Instrument médical selon la revendication 12, **caractérisé en ce que** le dispositif de blocage (13) est réalisé en tant que verrouillage à excentrique dans la zone du dispositif de serrage (7).

15. Instrument médical selon l'une au moins des revendications 5 à 14, **caractérisé en ce que** le montage à mobilité de rotation de l'élément de couplage (4) dans le dispositif de serrage (7) peut être limité par l'intermédiaire d'une goupille de verrouillage (14).

16. Instrument médical selon l'une au moins des revendications 1 à 15, comprenant un outil placé à l'extrémité distale du corps d'instrument en tige (1) et pouvant être actionné par l'intermédiaire de la poignée de préhension (3), **caractérisé en ce que** l'outil (2) peut être actionné par l'intermédiaire des poignées de manoeuvre (5) de la poignée de préhension (3), la poignée de préhension (3) et l'outil (2) étant reliés mutuellement par l'intermédiaire d'au moins un dispositif de transmission de force (16).

17. Instrument médical selon la revendication 16, **caractérisé en ce que** ledit au moins un dispositif de transmission de force (16) est réalisé en tant qu'élément de transmission de force flexible, notamment sous forme de câble sous gaine (17) appelé système de transmission Bowden.

18. Instrument médical selon la revendication 16, **caractérisé en ce que** ledit au moins un dispositif de transmission de force (16) fonctionne par voie hydraulique.

19. Instrument médical selon l'une au moins des revendications 16 à 18, **caractérisé en ce qu'**en plus de l'actionnement de l'outil (2) par l'intermédiaire des poignées de manoeuvre (5) de la poignée de préhension (3), il est également possible d'actionner le dispositif de serrage (7) par l'intermédiaire des poignées de manoeuvre (5) de la poignée de préhension (3).

20. Instrument médical selon la revendication 19, **caractérisé en ce que** l'actionnement du dispositif de serrage (7) par l'intermédiaire des poignées de manoeuvre (5) de la poignée de préhension (3), peut être découplé de l'actionnement de l'outil (2) par l'intermédiaire des poignées de manoeuvre (5) de la poignée de préhension (3).

21. Instrument médical selon la revendication 20, **caractérisé en ce que** les deux fonctions d'actionnement pouvant être exercées par l'intermédiaire des poignées de manoeuvre (5) de la poignée de préhension (3), peuvent être découplées de façon telle, que lors de la sélection d'une fonction d'actionnement, l'autre fonction d'actionnement soit automatiquement bloquée.

22. Instrument médical selon la revendication 21, **caractérisé en ce que** sur la poignée de préhension (3) est placé un dispositif de commutation pour établir la fonction d'actionnement pouvant être exercée par l'intermédiaire des poignées de manoeuvre (5) de la poignée de préhension (3).
